# EUROPEAN PATENT SPECIFICATION

(11) **EP 1 170 032 B1**
(45) Date of publication and mention of the grant of the patent: **27.10.2004**
(21) Application number: 00850144.7
(22) Date of filing: 28.08.2000
(51) Int. Cl.: A61N 2/02

(54) **Magnetic field generator for an electromedical treatment apparatus**
Magnetfeldgenerator für eine elektromedizinische Behandlungsvorrichtung
Générateur de champ magnétique pour un apparaeil de traitement électromédical

(30) Priority: 07.07.2000 SE 0002568
(43) Date of publication of application: 09.01.2002
(73) Proprietor: Med-Tech A/S, 9881 Bindslev (DK)
(72) Inventor: Lindholt, Claus, 9700 Brönderslev (DK)
(74) Representative: Hammond, Andrew David

(56) References cited:
- EP-A- 0 383 457
- EP-A- 0 453 602
- EP-B- 0 062 032
- US-A- 3 915 151

## Description

### TECHNICAL FIELD

The invention relates to a magnetic field generator for an electromedical treatment apparatus according to the preamble of claim 1 and 10. The invention further relates to a field generator for an electromedical treatment apparatus according to the preamble of claim 19. Still further the invention relates to an electromagnetic treatment apparatus for the treatment of biological tissue, preferably for accelerating the healing process in a part of the human body like an arm or leg, comprising a first and a second magnetic generator. Finally the invention relates to a method of producing a field generator for an electromedical treatment apparatus for treatment of biological tissue.

### BACKGROUND OF THE INVENTION

A large part of the economic and personnel resources of medical service is used for the treatment of patients with long lasting illness periods. As an example we can mention the treatment of fractures such as fractured thigh bone, treatment of rheumatism and patients with neurologic movement handicaps and long term therapy patients with slow healing infected wounds etc. For this reason it is not least of economical value, but also reducing suffering of great importance to point out and use medical method and equipment that accelerate and support the healing processes.

It has been known for a long time that magnetic and electric fields affect biological tissue. Injuries and diseases have been treated with varying results. Magnetic and electrical fields have been applied against the diseased and wounded body part and one has hoped for the best. An example of this kind of treatment is described in US patent number 3,915,151. It is primarily adapted for treatment of broken bones and uses preferably flat coils for generation of a magnetic field whose flow is arranged longitudinally with the treatment object, i.e. parallel to a leg or an arm. In one embodiment an electrostatic field is introduced besides the magnetic field. This is achieved by two diametrically opposite electrodes, to which a voltage with relatively high potential difference is supplied. An electric field is hereby achieved which flows through the treatment object essentially at right angles to the flow of the magnetic field. By supplying a voltage, whose amplitude fluctuate regularly, the electrically charged particles in the treatment area are brought into an oscillating movement. Due to the flow directions of one magnetic and one electric fields, the charged particles oscillate only at right angles to the skeletal structure and thus perpendicular to the main blood flow of the treatment object. The venous blood flow, i.e. the blood flow towards the heart, is thereby not effected by this treatment.

Another apparatus is described in GB-A-871672. This document describes an apparatus for medical purposes, in which an electric and a magnetic field are produced. These field are adjustable and variable and can be changed in polarity. The electric and magnetic fields are disposed at right angles to each other to produce in the object treated by their simultaneous action an electrodynamic effect which can be brought to an optimum value by the adjustment or variation of the phase relation of the two fields. One application of this apparatus is a shortwave apparatus, which produces at the same time an electrical capacitor field and a magnetic coil field in a region, which can be selected as desired in the object treated. This apparatus is fed by alternating current, which means that the electrolyte will rotate in two different directions owing to the direction of the magnetic field. This means that the electrolyte rotates against the flow direction of the blood in half the cycle and thereby counteracts against the circulation of the blood. This also results in that heat is generated in the electrolyte.

In order to resolve the problems mentioned above an electromagnetic treatment apparatus as described in EP-62032 was introduced. By using the apparatus as disclosed in EP-62032 a device that accelerated the healing process by bringing electrically charged particles in tissue to rotate in a helix shaped movement which support the metabolism in the tissue and increases the volume of the blood flow. The device is used for treatment of patients suffering of rheumatics as well as treatment of patients with circulatory disturbances and thrombosis. The electrical and magnetic field that produces the helix shaped movement is obtained by arranging magnetic and electric lenses inside and coaxially with solenoids providing the main magnetic field.

It has, however shown that the device as disclosed in EP-062032 is difficult to manufacture and that the device is primarily intended for manual manufacturing. This has made the product more expensive and thereby has caused the device to be less successful on the market. Furthermore, the manual and complicated manufacturing process have increased the risk for misplacement of lens elements in the device. Such misplacement could severely deteriorate the function of the device. Especially the use of core elements for the magnetic lens elements that are attached to the main core of the magnetic field generator has resulted in a difficult manufacturing process.

### SUMMARY OF THE INVENTION

An object of the invention is to provide a magnetic field generator, which is designed for automatic manufacturing and thereby safeguarding against misplacement of lens elements in the device whereby risk for production of deteriorated magnetic field generators is reduced.

This object is achieved by a magnetic field generator having lens core elements with two free ends, that is lens core elements not attached to the main core, according to the characterising portion of claim 1. By providing a magnetic field generator that includes a support element for the magnetic lenses arranged within said opening of the main core producing a main magnetic field the risk for misplacement of the lens elements are reduced, especially since the lens cores are not attached to the main core.

The object of the invention is also achieved by a magnetic field generator according to the characterising portion of claim 10. By providing lens solenoids arranged on the lens core elements each have two free wire connections each connected to a circuit card supporting said main core the magnetic field generator is adapted for automatic use and the correct position of lens elements can be safeguarded.

A second object of the invention is to provide a field generator for an electromedical treatment apparatus, which is designed for automatic manufacturing and thereby safeguarding against misplacement of lens elements in the device whereby risk for production of deteriorated field generators is reduced.

This object is achieved by a field generator having lens core elements with two free ends, that is lens core elements not attached to the main core, according to the characterising portion of claim 19. By providing the electric lens elements on a circuit card supporting said main core the risk for misplacement of the electric lens elements is reduced and the field generator is adapted for industrial production.

A third object of the invention is to provide a production method for field generators, including magnetic field generators having magnetic field elements with lens cores that are not attached to the main core, where the risk for misplacement of the magnetic and/or electric lenses is reduced. This is achieved by a method according to claim 30. By first connecting magnetic lenses, a support for magnetic lenses and a main core element to a circuit card and thereafter arranging a main solenoid on said main core, the magnetic lenses are supported by the support element, the main core, the circuit card and the main coil and thereby prevented from accidental movement to an incorrect position.

A fourth object of the invention is to provide an electromagnetic treatment apparatus for the treatment of biological tissue, preferably for accelerating the healing process in a part of the human body like an arm or leg, comprising a first and a second magnetic generator having the advantages of the field generator or magnetic field generator according to the invention. This object is achieved by an electromagnetic treatment apparatus according to claim 29.

### DESCRIPTION OF DRAWINGS

An embodiment of the invention will be described below with reference to the attached drawings, where
- fig. 1: is a sideview in perspective of a prior art electromedical treatment apparatus,
- fig. 2: show an electrical circuit diagram of the apparatus,
- fig. 3: show an exploded view of a field generator according to the invention,
- fig. 4: show a top view of field generator according to the invention,
- fig. 5a-5c: show some examples of particle paths in electromagnetic fields,
- fig. 6: show a cross section of a field generator according to the invention along the section A - A in fig 4, and
- fig. 7: show a view from below of a support element.

### DETAILED DESCRIPTION OF THE INVENTION

### Prior art

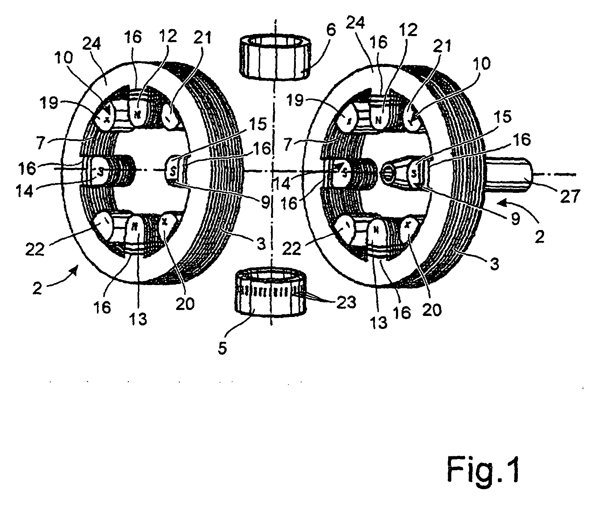

In figure 1 a prior art field generator is shown. Since the prior art field generator contains similar elements and generates a similar electric and magnetic field the prior art field generator will be described in detail. In fig. 1 there is shown a schematic side view of a prior art treatment apparatus. The parts included are two ringshaped solenoid systems 2 coaxially arranged horizontally and at some distance from one another. Each solenoid system 2 is at its circumference equipped with a solenoid 3. These solenoids are electrically connected in series and form together a magnetic field generator system 4. Between the two solenoid systems 2 there is a lower and an upper field electrode 5,6 arranged in a way, that the central axis in each field electrode coincide with each other. The common central axis of the field electrodes 5,6 intersects the central axis of the solenoid system 2 perpendicular and at essentially the same distance from each solenoid system 2. The solenoid systems 2 and the field electrodes 5,6 are arranged in a way that each of them can manually be adjusted along a horizontal resp. vertical direction with respect to each other. This arrangement results in that the centre of the treatment apparatus i.e. the treatment zone between the solenoid system 2 and the field electrodes 5,6, which is defined by the region where the magnetic and electric fields cross, can be adjusted to larger or smaller size and in this way be adapted to the size of the object to be treated, for example it cam be smaller if an object such as an ankle is to be treated, or larger when a femur is to be treated.

Each of the solenoid systems 2 included in the treatment apparatus consists of a ringshaped core 7, built up of transformer core sheets 8. These sheet metal lamina of for example iron, are in a conventional way isolated from each other and tightly packed in the longitudinal axial direction of the solenoid system 2. In the defined inner space of each ringshaped core 7 is arranged one magnetic and one electric lens 9,10. The elements 12- 15, 19 - 22 included in each lens are attached to the inner surface of the ringshaped core 7.

At the circumference of the solenoid system 2, on the outer surface of the ringshaped core 7 there is arranged a solenoid 7. The solenoid 3 consists of an isolated copper coiling whose wire diameter is adjusted to the maximum current which is to be fed through the solenoid 3. To keep the coils of the solenoids 3 in place two side plates 24 of dielectric material are arranged at each side surfaces of the solenoids. In order to maintain a sufficient strong magnetic field a number of wiring turns may be necessary. Both of the solenoids 3 included in the treatment apparatus are electrically connected in series whereby a homogenous magnetic field is maintained in the treatment zone.

The magnetic lens 9 arranged in the core 7 is of the so called quadrupole type and contains four lens elements 12 - 15 each of which forms a magnetic pole. The preferably cylindrically shaped lens elements 12 - 15, which for example can be punched out from the same material as the ringshaped core 7 are located symmetrically along the inner surface of the core 7 in such a way that the two lens elements 12, 13 which forms the north poles and the two lens elements 14, 15 which forms south poles are arrange in pairs opposite each other. The surface of the lens element 12 - 15 turned inwardly towards the centre of the magnetic lens 9 are hyperbolically formed. Hereby a more homogenous magnetic field in the centre of the magnetic lens is maintained. Around each lens element 12 - 15 there are arranged lens solenoids 16 in such a manner that the central axis of each solenoid 16 coincides within one and the same point in the centre of the solenoid system 2.

The lens solenoids 16 are connected electrically in a way that two diametrically arranged lens elements 14,15 work as south poles and the other two as north poles. The lens solenoids 16 included in the magnetic lens can be electrically connected in series and connected to a voltage unit 17. The main purpose of the magnetic lens 9 is to converge electrically charged particles which pass through the magnetic lens 9. Its function should, however, be seen in relation to other included parts of the electromagnetic treatment apparatus 1.

Also the electric lens 10 included in the solenoid system 2 is of the so called quadrupole type, i.e. also this has four lens elements here called lens electrodes 19 - 22, Each lens electrode 19 - 22 consists of an electrically conducting material, for example copper or the like, and is essentially rod shaped. The surface facing the centre of the solenoid system is preferably hyperbolically shaped in order to maintain the desired field distribution. The lens electrodes 19 - 22 are symmetrically arranged inside the ringshaped core 7 of the solenoid systems 2, in such a way that the two lens electrodes 19,20 with positive potential and the two lens electrodes 21, 22 with negative potential are arranged in pairs diametrically opposite to each other. The lens electrodes 19 - 22 that are electrically insulated from the ringshaped core with insulators 25 are displaced by an angle of 45° relative to the lens elements 12 - 15 contained in the solenoid system 2. The lens electrodes 21 - 22 that have a negative potential is connected and wired to the negative terminal of the voltage unit 17. The above described electrical lens 10 diverges the electrically charged particles that pass through its central section.

In the middle of the electromagnetic treatment apparatus, i.e. in its treatment zone, there is arranged an electric field which is directed perpendicular to the magnetic field. This electrical field is generated by an upper and a lower field electrode 6,5 constituting part of an electrical field generator 26. The upper field electrode 6 consists of a ringshaped pipe of metal whereon the pipe end turned towards the treatment zone is hyperbolically shaped. This upper field electrode 6 is supplied with a positive potential, which results in that the flow of the electrical filed is directed downwards.

The lower field electrode 5 consists in the same way as the upper field electrode of metal and is formed as a ringshaped pipe. A grid 23 is arranged at its inner edge. The grid 23 consists of bands or rods of tungsten or molybdenium and are arranged in the upper part of the lower field electrode 5. The upper edges of the grid 23 are formed with a sharp egg-shaped profile and directed towards the upper field electrode 6. Hereby an electric field is obtained, whose flow is focussed toward the.inner part of the lower field electrode. This results in that a more concentrated field distribution is obtained in the treatment zone.

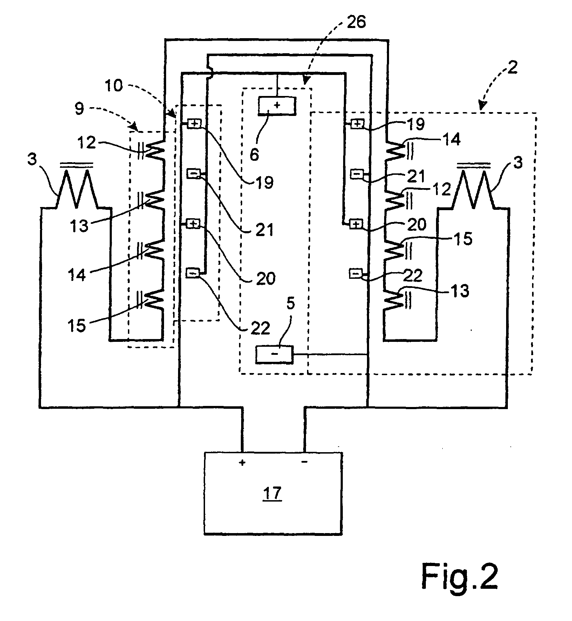

As is shown in fig. 2 all solenoids 16 arranged at the lens elements 12 - 15 are connected in series and all lens electrodes 19, 20 with positive potential are connected together and wired to the positive terminal of the voltage unit 17. The lens electrodes 21, 22 are in a related way connected to the negative terminal of the voltage unit 17.
One and the same voltage unit supply the entire treatment apparatus 1 with is electrical energy. The voltage consists of a half wave-rectified sinus voltage that never passes zero. The frequency with which the voltage varies has been chosen to 66 Hz, but can naturally vary between for example 20 and 100 Hz. Also the amplitude of the voltage is freely adjustable. The voltage to the solenoids 3 and 16 can either be positive or negative. It is of course possible to separate the purely electrical system from the electromagnetic system and thereby supply the solenoids 3, 16 on one hand and the lens electrodes 19 - 22 and the field electrodes 5,6 on the other hand with voltages whose values differ. It is, however, desirable that the voltage to both systems can vary in accordance to amplitude frequency and polarity.

In one embodiment a pipe or a nozzle 27 for gas or liquid additive can be arranged at one or both ends of the electromagnetic apparatus, i.e. coaxially with the central axis of solenoid system 2. Through this pipe or nozzle 27 electrically charged particles possibly mixed with one or more medically active substances can be supplied to the treatment zone via the solenoid systems.

### Short Theoretical Magnetohydralical Background

The function of electromagnetic treatment apparatus is based on generally accepted physical and chemical laws as well as discoveries within the medical research. The theoretical basis is among others collected from the magnetohydrodynamics and the plasmaphysics disclosed in "Classical Electrodynamics" by Jackson and "Vacuumgaselectronphysics" by Lyman Spitzen, Princeton University. What connects to the medical discoveries reference is mad to for example Medicine and Biology, No 3, 1969.

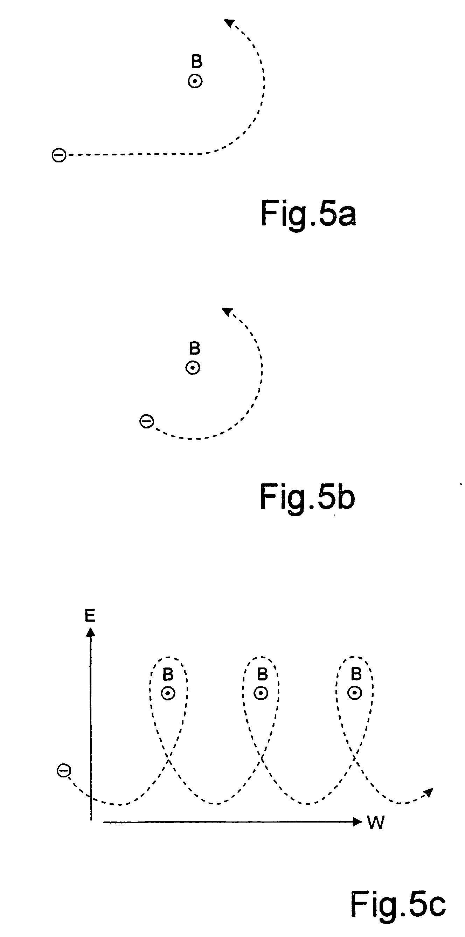

Fig. 5a - 5c shows paths of charged particles in different fields according to accepted laws of nature. E and B represent an electric and a magnetic field. In fig 5a is shown the movement path of a positive particle in a magnetic field directed "away from the viewer". In fig. 5b is shown the movement path of a negative particle in a magnetic field directed "toward the viewer". Finally in fig 5c is shown how a negatively charged particle performs a helically shaped movement in two against each other perpendicular fields, whereby the particle is transported over a distance W. A magnetic field B directed toward the viewer and an electric field E directed upward (as shown by arrows). It is among other things this movement that is achieved by electromagnetic treatment apparatus described in EP 062032 and electromagnetic treatment apparatus according to the invention.

### Function of electromagnetic treatment apparatus

Our blood, as is known, contains a number of different elements and substances. The most common ions, the so called anions and cathions are potassium-, sodium-, chloride-, calcium-, iron-, sulphate-, and phosphate ions. When an ion is a particle whose resultant charge is either positive or negative, it is influenced by electric and magnetic fields. If the ion passes through one of these fields energy is transferred to it whereby it moves along paths as shown in fig 5a - 5c. The velocity of the ion depends among other factors on the viscosity of the surrounding substance. Also the ability of the ion to drag with it other particles in the substance can effect the mobility. Convection movements in the substance and density gradients can to some extent also affect the movement of the ion.

In a number of injuries and diseases it has been advantageous to use a treatment that causes hyperaemia, i.e. localised oversupply of blood and increased throughflow of blood. This has so far been achieved by physiotheraphy, whereby mainly different forms of heating apparatus have been used, such as heating lamps and high frequency techniques. The increased blood flow which has been achieved in this way, assists the transportation of e.g. nutritional elements, proteins and oxygen to the cells and undissolved products from them. This metabolism supports the healing processes. A substantial drawback during treatment with heating lamps is that the infrared radiation can only pass through a few millimetres in the skin and thereby only effects the body surface.

An electromedical treatment apparatus according to the invention or EP 062032 is effective also on deeper lying tissue. This is due to that all positive or negative charged particles in an electric or magnetic field are affected and that these particles are spread in all parts of the tissue.

The electromagnetic radiation is intended to affect the bioelectrical potentials on the cellular plane of the organism. Cell membranes and mitochondria etc. but naturally also will affect water distribution in the organism depending on the specific structure of the water molecules. Other organic molecules with dipole characteristic will probably also be affected. When voltage is supplied the treatment apparatus generates a horizontal magnetic field and a vertical electric field defined by the region where the magnetic and electric fields cross. If charges particles, for example the ions in the blood plasma, are supplied into the treatment zone they are influenced to move in a determined pattern. The path of the ions is influenced by the strength of the magnetic and electric fields. They drag with them uncharged particles in their immediate surroundings. The strength of the fields is by choice adjustable. The magnetic flux density reaches for example 2000 Gauss. Both the strength of the magnetic and electric fields can vary at the rate of the pulse frequency of the introduced voltage. A pulse frequency of 66 Hz has been shown to coincide with the biological activity of the body but can, when needed, be adjusted between 20 - 80 Hz.

By locating the relevant bodypart, for example an arm or leg, in the treatment zone substantially perpendicular to both the magnetic and the electric field, the ions perform a helically formed movement in the direction of the length of the blood vessels (see fig 5c). When substantially all charged particles have an equal translatory movement component and each charged particle to a greater or lesser extent drags with it other, uncharged closely lying particles, an increased through flow of blood in the vessels is obtained.

The energy transferred to the ion results in a movement, which in short can be described in the following way. A positively charged particle that approaches a magnetic field will curve and rotate counter clockwise seen in a view of the direction of the flow of the magnetic field. If an electric field is applied perpendicular to the magnetic field and the electric field is directed upward, the positively charged particles will accelerate onto the right side of the magnetic field. If the particle is negatively charged, the relationship will be the opposite. The final movement path will be helixshaped, i.e. screwformed, with a constant pitch. This is visualised in fig. 5c.

Due to the fact that the charged particles start to move and thereby bring with them close lying particles, an advantageous increase of the blood through flow is obtained in the treated tissue. Also an analgesic, i.e. pain reducing, effect can be shown. Pain depends on lack of oxygen in the tissue or on an accumulation of pain substances. An increase of the blood flow gives increased oxygen supply and supports the removal of the pain substances.

Since also the central nervous system works by means of electrically charged particles which among others pass in an out of the nerve cells an in this way transmit information, to for example muscles, also neurologically caused wounds or diseases can be treated with the electromagnetic apparatus described herein.

A large part of diseases for older person stem from the circulatory system and its peripheries. Within this disease area it is difficult to produce adequate medicine because the side effect on other organs increase, especially on older persons. Many persons suffer from so called calcification of the skeleton when they get older. This greatly increases the risk of fractures, where femur breakage is a common injury. The treatment period for this type of injury can be very long and consume a large part of medical service resources. With the treatment of the elctromagnetic apparatus described herein a quicker healing is achieved when treating these breaks. It has been shown that calcium ions can be forced to wander back into the skeleton whereby a delimited skeleton can be regenerated. In this way bone fractures can be prevented.

The healing of leg wounds, burns and slowhealing, infected sores can also be accelerated with the electromagnetic apparatus described herein. This can for example be carried out by the fact that a medically active substance such as active iodine, or antibiotics in the form of a spray is supplied to the treatment zone by acceleration from one or both of the solenoid systems 2 via for example a nozzle 27. This is also the case for hyperbar oxygentherapy. A bombardment of the wound with medically active substances can thereby be achieved. Due to this method of treatment otherwise developing resistant infection causes can be avoided.

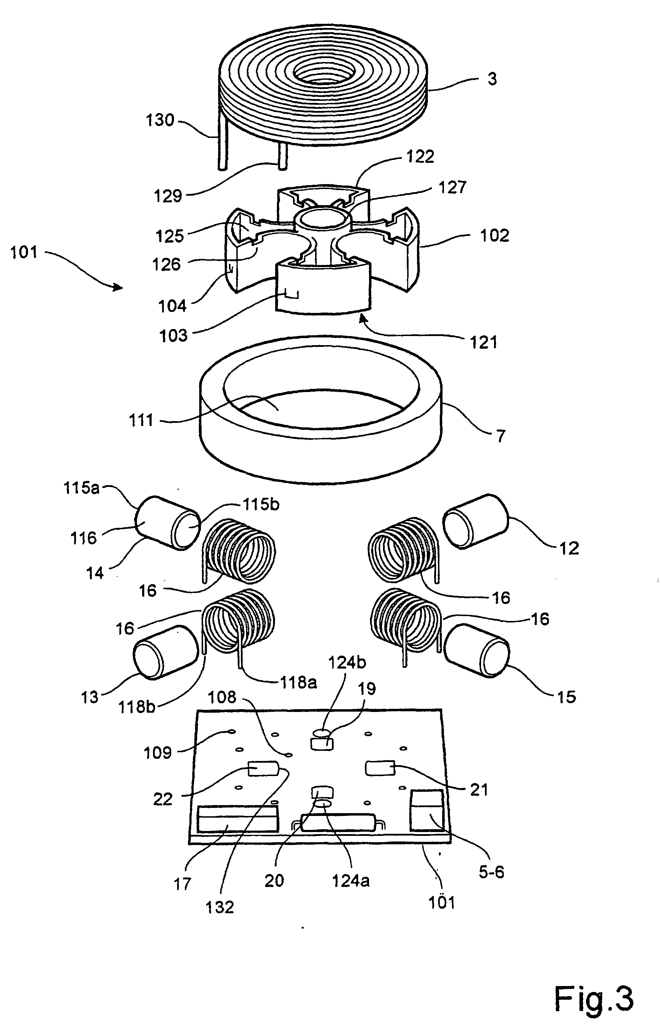

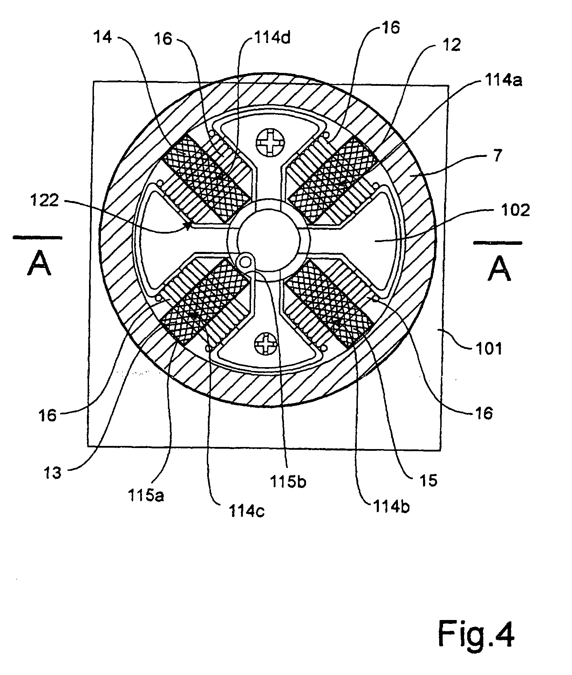

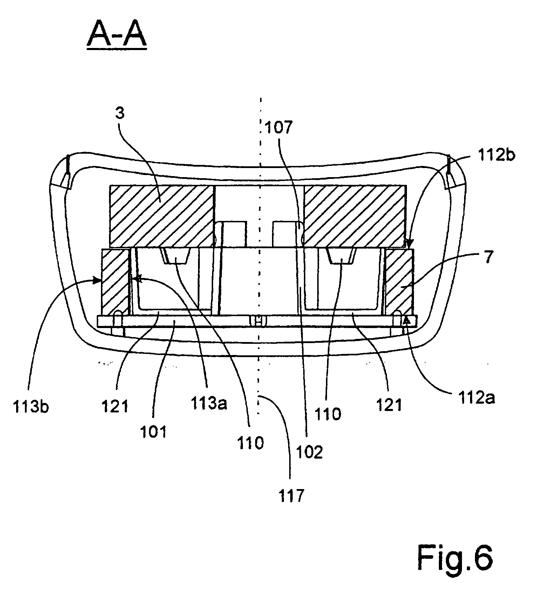

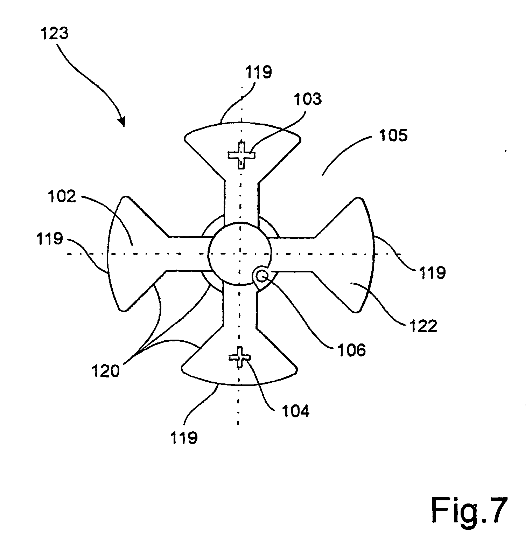

The invention will be further described with references to Fig. 3, which shows an exploded view of a field generator for an electromedical treatment apparatus according to the invention and Fig. 4 which show a top view of a field generator according to the invention, Fig. 6 which show a cross section of a field generator according to the invention along the section A - A in fig 4 and Fig.7, which show a view from below of a support element for the magnetic lenses, the main core and the main coil. The field generator according to the invention can preferably be used in an electromedical treatment apparatus of the prior art type described in relation to figure 1.The field generator includes a magnetic field generator and an electric field generator.

The magnetic field generator includes at least one solenoid 3 arranged on a main core 7 defining an opening 111. The main core 7 has first and second axial wall portions 112a, 112b wherein said opening 111 is defined and inner and outer radial wall portions 113a, 113b. The main core 7 preferably defines a closed magnetic circuit and is in a preferred embodiment arranged as ring- or toroid shaped core. In a preferred embodiment of the invention the solenoid 3 is arranged on one of said axial wall portions 112a, 112b and preferably partly covering the opening 111 in the main core 7. By this arrangement the solenoid 3 would constitute support for magnetic lens elements 114a -d arranged inside an opening or channel 111 in the main core 7 defined by said openings 111 and the inner radial wall 113a of the main core 7.

The magnetic field generator includes a magnetic lens system. The lens system preferably includes an even number of lens elements 114a - d arranged inside said opening or channel of said main core. Each magnetic lens 114 a - d includes a lens core element 12 - 15 having two free ends 115 a, 115b, i.e. is not attached to the main core 7 and a solenoid 16 arranged on said lens core element 12 - 15. The lens core 12 - 15 is preferably pinshaped, i.e. shaped as a elongated cylindrical element having two end portions 115a, 115b and a envelope surface 116. The lens cores 12 - 15 are preferably substantially vertically arranged in relation to the inner wall 113a of the main core 7 and arranged substantially parallel with a plane extending perpendicularly to a central axis of said channel or opening 111 of said main core 7. The end portions 115a of the lens cores, directed towards the central axis 117 of the channel or opening of said main core 7, are preferably hyperbolically shaped. Each lens core 12 - 15 carries a lens solenoid 16 arranged on the lens cores. Each lens solenoid 16 has two free wire connections 118a, 118b each connected to a circuit card 101. In a preferred embodiment of the invention the circuit card 101 supports the main core 7 as well as the lens solenoids 16. In a preferred embodiment of the invention the lens cores 12 - 15 are axially moveably arranged within the lens solenoids 16 and can therefore, after the magnetic lens elements 114a - d and the main core 7 has been arranged on the circuit card 101, slide towards the main core 7 whereby the lens cores 12 -15 make contact with the main core 7.

The magnetic lens elements 114a - d are supported by a support element 102 arranged inside the channel or opening 111 of the main core 7. The support element 102 is made of a nonmagnetic and non-conducting material, for example of a plastic material. The support element 102 has a first set 119 of support surfaces arranged for preventing movement of the main core in a radial direction furthermore the support element has a second set 120 of support surfaces arranged for preventing dislocation of the magnetic lens elements and in particular for preventing dislocation of the lens cores slidably arranged in the lens solenoids. Furthermore the support element in a preferred embodiment includes a third set 121 of support surfaces arranged for supporting a circuit card and in a still further preferred embodiment a fourth set 122 of support surfaces arranged for supporting the main solenoid. In a preferred embodiment where the opening or the channel 111 of the main core 7 is cylindrical, the support element 102 is substantially shaped as a cylinder having recesses forming 123 said second set 120 of support surfaces. The radially outwardly directed envelope surface constitutes said first set 119 of supporting surfaces and parts of a first and a second axial end walls constitutes said third and fourth set 119, 120 of supporting surfaces. In a preferred embodiment, as seen in fig 3., an axial cross section of the support element 102 is of the shape of a Maltese cross, where the gaps between the wings of the cross constitutes support for the lens elements and the radially outwardly directed parts of the wings constitutes support for preventing dislocation of the main core. The end wall 121 constituting support for a circuit card 101, is in a preferred embodiment provided with attachment means 103, 104 for attaching to the circuit card. In a preferred embodiment the attachment means 103, 104 are in form of a set of knobs adapted for a snug fit in holes 124a, 124b arranged on the circuit card. The support element 102 could be arranged as a solid body or, as shown in fig 3, encompass cavities 125. The cavities 125 could be open or closed. In a preferred embodiment of the invention the support element 102 includes distribution channels 126 for facilitating access for injection of a solidifying compound. The compound is injected after assembling the field generator for stabilising the generator and preventing dislocation of any of its parts. In the embodiment shown in fig. 3 the distribution channels 126 are formed as recesses provided in the second set 120 of support surfaces giving support to the lens elements. Furthermore the fourth set 122 of supporting surfaces includes in a preferred embodiment means 127 for preventing radial dislocation of the main coil 3. In the embodiment shown in fig. 3 these means 127 are formed as an axial protrusion on the fourth set 122 of supporting surfaces of the support element. The protrusion 127 is preferably formed for a snug fit in a central opening 128 of a toroid shaped main solenoid 3. The support element 102 thereby gives axial and radial support for the main solenoid 3. Furthermore the support element 102 could be provided with a narrow connection channel 106 for an end wire 129 of the main solenoid. The connection channel retains the wire and therefore even further stabilises the field generator and facilitates connection for the free end to the circuit card. The other free wire end 130 of the main solenoid is preferably connected to the same circuit card 101, preferably in a connection radially outside the main core.

The circuit card 101, which is of conventional type, includes connections 131a,b for the free wire ends of the lens solenoids. The circuit card further includes connection 17 for voltage supply to the lens solenoids and preferably also for the main solenoid. The circuit card also includes electrical conductors (not shown), formed by etching according to normal practice, connecting opposite pairs of magnetic south poles and opposite pairs of magnetic north poles. The magnetic lens elements preferably constitute a quadrupol element having two diametrically opposed south poles and two diametrically opposed south poles. The southpoles are arranged at 90° from each other. The circuit card furthermore, in a preferred embodiment includes an even number of electrical lens elements 19 - 22, preferably four, forming part of an electric lens system in an electric field generator. The lens elements 19 - 22 are arranged radially within the opening of the main core. The electrical lens elements are formed as plate shaped electrodes arranged on or in the circuit card. The electrical lens elements are preferably hyperbolically shaped in the region 132 directed towards the centre of the opening of the main core. The circuit card furthermore includes conductors connecting the electrical lens elements in pairs of positive potential and pairs of negative potential where each electrode in a pair is arranged at substantially diametrically opposed positions. In a preferred embodiment of the invention the electrical lens system is of quadrupol type and includes four lens elements, two intended to have negative potential arranged at diametrically opposite positions and two lens elements intended to have positive potential arranged at diametrically opposite positions, where the negative and the positive electrodes are arranged 90° from each other. In a preferred embodiment the electrical lenses are arranged at 45° from the positions of the magnetic lenses.

In a preferred embodiment of the invention the circuit card is provided with attachment means for attaching the circuit card to a housing. In a preferred embodiment the attachment means are formed as knobs arranged on the inside of the housing which are arranged for being received in holes arranged on the circuit card.

The invention also relates to a method of producing a field generator for an electromedical treatment apparatus for treatment of biological tissue. The method involves a series of method steps.

In a first step the magnetic lenses, the support element and the main core is attached to a circuit card. In a preferred embodiment free ends of solenoids of the magnetic lens elements are attached to connections provided at the circuit card. After positioning the solenoids at the circuit card, the support element is attached to the circuit card thereby immediately stabilising the lens elements. Thereafter the main core is assembled by positioning the main core around the support element. By that operation the lens core elements positioned inside the lens solenoids are prevented from being dislocated.
At a second method step performed after attaching the lens solenoids, the support element and the main core the main solenoid is arranged on the main core. In a preferred embodiment the main solenoid at least partly covers the opening in the main core and thereby together with the main core, the support element and the circuit card encloses the lens solenoids.

In a preferred embodiment the circuit card is thereafter attached to an inside wall of a housing by attachment means. Furthermore, in a preferred embodiment, a solidifying compound is injected into the housing after the assembly of the parts of the field generator has been completed thereby still further stabilising the parts of the field generator from being dislocated. Epoxy could be used as solidifying compound.

The invention should not be limited to the examples set out above, but could be varied within the scope of the claims

## Claims

1. Magnetic field generator for an electromedical treatment apparatus for treatment of biological tissue incorporating at least one solenoid (3) and a main core (7), the solenoid being substantially coaxially arranged with an opening (111) defined in said main core and a magnetic lens system including an even number of lens elements (114a-d) arranged inside said opening of said main core, where each magnetic lens includes a lens core element (12-15) having two free ends (115a, 115b) and a lens solenoid (16) arranged on said lens core element **characterised in that** said magnetic field generator further includes a support element (102) for the magnetic lenses arranged within said opening of said main core.

2. Magnetic field generator according to claim 1, **characterised in that** said support element is centrally arranged within said opening of said main core.

3. Magnetic field generator according to claim 1 or 2, **characterised in that** said support element includes wall portions forming a recess preventing dislocation of the lens solenoids

4. Magnetic field generator according to any of claims 1 - 3, **characterised in that** said support element includes wall portions forming a recess preventing dislocation of the lens core in a direction towards a centre of the opening of said main core

5. Magnetic field generator according to any of claims 1 - 4, **characterised in that** said recess together with the main core element encloses each lens element and prevents each lens element from dislocation in a plane extending through the main core element.

6. Magnetic field generator according to any of claims 1 - 5, **characterised in that** said support element includes distribution channels for providing access for injection of a solidifying compound.

7. Magnetic field generator according to any of claims 1 - 6, **characterised in that** said support element is arranged for supporting a circuit card supporting said main core.

8. Magnetic field generator according to any of claims 1 - 7, **characterised in that** said support element provides support for the main coil.

9. Magnetic field generator according to any of claims 1 - 8, **characterised in that** each lens solenoid arranged on said lens core element have two free wire connections each connected to a circuit card supporting said main core.

10. Magnetic field generator for an electromedical treatment apparatus for treatment of biological tissue incorporating at least one solenoid (3) and a main core (7), the solenoid being arranged on said main core defining an opening (111) and a magnetic lens system including an even number of lens elements (114a-d) arranged inside said opening of said main core, where each magnetic lens includes a lens core element having two free ends (115a, 115b) and a lens solenoid arranged on said lens core element **characterised in that** the lens solenoids arranged on the lens core elements each have two free wire connections (118a, 118b) each connected to a circuit card (101) supporting said main core.

11. Magnetic field generator according to claim 10, **characterised in that** said magnetic field generator further includes a support element for the magnetic lenses arranged within said opening of said main core.

12. Magnetic field generator according to claim 11, **characterised in that** said support element is centrally arranged within said opening of said main core.

13. Magnetic field generator according to any of claims 11 - 12, **characterised in that** said support element includes wall portions forming a recess preventing dislocation of the lens solenoids

14. Magnetic field generator according to any of claims 11 - 13, **characterised in that** said support element includes wall portions forming a recess preventing dislocation of the lens core in a direction towards a centre of the opening of said main core

15. Magnetic field generator according to any of claims 11 - 14, **characterised in that** said recess together with the main core element encloses each lens element and prevents each lens element from dislocation in a plane extending through the main core element.

16. Magnetic field generator according to any of claims 11 - 15, **characterised in that** said support element includes distribution channels for providing access for injection of a solidifying compound.

17. Magnetic field generator according to any of claims 11 - 16, **characterised in that** said support element is arranged on to a circuit card supporting said main core.

18. Magnetic field generator according to any of claims 11 - 17, **characterised in that** said support element provides central support for the main coil.

19. Field generator for an electromedical treatment apparatus for treatment of biological tissue including a magnetic field generator having at least one solenoid (3) and a main core, the solenoid being arranged on said main core defining an opening (111) and a magnetic lens system including an even number of lens elements (114 a-d) arranged inside said opening of said main core, where each magnetic lens includes a lens core element (12-15) having two free ends (115a, 115b) and a solenoid 16 arranged on said lens core element and an electric field generator having an electric lens system including an even number of lens elements (19-22) arranged radially within the opening of said main core **characterised in that** the electric lens elements are arranged on a circuit card (101) supporting said main core.

20. Field generator according to claim 19, **characterised in that** said field generator further includes a support element for the magnetic lenses arranged within said opening of said main core.

21. Field generator according to claim 20, **characterised in that** said field generator further includes a support element for the magnetic lenses arranged within said opening of said main core.

22. Field generator according to claim 20 or 21, **characterised in that** said support element is centrally arranged within said opening of said main core.

23. Field generator according to any of claims 20 - 22, **characterised in that** said support element includes wall portions forming a recess preventing dislocation of the lens solenoids

24. Field generator according to any of claims 20 - 23, **characterised in that** said support element includes wall portions forming a recess preventing dislocation of the lens core in a direction towards a centre of the opening of said main core

25. Field generator according to any of claims 20 - 24, **characterised in that** said recess together with the main core element encloses each lens element and prevents each lens element from dislocation in a plane extending through the main core element.

26. Field generator according to any of claims 20 - 25, **characterised in that** said support element includes distribution channels for providing access for injection of a solidifying compound.

27. Field generator according to any of claims 20 - 26, **characterised in that** said support element is arranged on to a circuit card supporting said main core.

28. Field generator according to any of claims 20 - 27, **characterised in that** said support element provides central support for the main coil.

29. Electromagnetic treatment apparatus for the treatment of biological tissue, preferably for accelerating the healing process in a part of the human body like an arm or leg, comprising a first and a second magnetic generator according to any of the preceding claims and that said first and second magnetic generators are arranged for providing a alternating magnetic field and that said electromagnetic treatment apparatus further includes a first and a second electrode arranged for generating an electric field perpendicular to said magnetic field.

30. Method of producing a field generator for an electromedical treatment apparatus for treatment of biological tissue according to any of the preceding claims comprising the following production steps:
- first connecting magnetic lenses, a support for magnetic lenses and a main core element to a circuit card;
- thereafter arranging a main solenoid on said main core.

31. Method of producing a field generator according to claim 30, **characterised in that** the solenoid provides axial support for the magnetic lens elements.

32. Method of producing a field generator according to claim 30 or 31, **characterised in that** the solenoid provides axial support for the magnetic lens elements.

33. Method of producing a field generator according to any of claims 30 - 32, **characterised in that** the field generator is enclosed in a housing.

34. Method of producing a field generator according to claim 33, **characterised in that** a solidifying compound in injected into the housing.

## Patentansprüche

1. Magnetfeldgenerator für eine elektromedizinische Behandlungsvorrichtung zum Behandeln von biologischem Gewebe, welcher mindestens eine Magnetspule (3) und einen Hauptkern (7) aufweist, wobei die Magnetspule in im Wesentlichen koaxialer Ausrichtung mit einer Öffnung (111) ausgebildet ist, die in dem Hauptkern definiert ist, sowie eine gerade Anzahl von Linsenelementen (116 a - d), die im Inneren der Öffnung in dem Hauptkern angeordnet sind, bei welchem jede magnetische Linse ein Linsenkernelement (12 - 15) mit zwei freien Enden (115a, 115b) sowie eine Linsen-Magnetspule (16) aufweist, die auf dem Linsenkernelement angeordnet ist,
**dadurch gekennzeichnet, dass** der Magnetfeldgenerator des Weiteren ein Stützelement (102) für die im Inneren der Öffnung in dem Hauptkern angeordneten magnetischen Linsen aufweist.

2. Magnetfeldgenerator nach Anspruch 1, **dadurch gekennzeichnet, dass** das Stützelement innerhalb der Öffnung in dem Hauptkern in der Mitte angeordnet ist.

3. Magnetfeldgenerator nach Anspruch 1 oder 2, **dadurch gekennzeichnet, dass** das Stützelement Wandungsabschnitte aufweist, die eine Vertiefung bilden, welche eine Verschiebung der Linsen-Magnetspulen verhindert.

4. Magnetfeldgenerator nach einem der Ansprüche 1 - 3, **dadurch gekennzeichnet, dass** das Stützelement Wandungsabschnitte aufweist, die eine Vertiefung bilden, welche eine Verschiebung des Linsenkerns in einer Richtung zur Mitte der Öffnung in dem Hauptkern hin verhindert.

5. Magnetfeldgenerator nach einem der Ansprüche 1 - 14, **dadurch gekennzeichnet, dass** die Vertiefung zusammen mit dem Hauptkernelement jedes Linsenelement umschließt und eine Verschiebung jedes Linsenelements in einer Ebene verhindert, die sich durch das Hauptkernelement hindurch erstreckt.

6. Magnetfeldgenerator nach einem der Ansprüche 1 - 5, **dadurch gekennzeichnet, dass** das Stützelement Verteilungskanäle aufweist, welche beim Einspritzen einer Verfestigungsmasse den Zugang schaffen.

7. Magnetfeldgenerator nach einem der Ansprüche 1 - 6, **dadurch gekennzeichnet, dass** das Stützelement so angeordnet ist, dass es eine Schaltungskarte als Träger des Hauptkerns abstützt.

8. Magnetfeldgenerator nach einem der Ansprüche 1 - 7, **dadurch gekennzeichnet, dass** das Stützelement für die Abstützung der Hauptspule sorgt.

9. Magnetfeldgenerator nach einem der Ansprüche 1 - 8, **dadurch gekennzeichnet, dass** jede Linsen-Magnetspule, die auf dem Linsen-Kernelement angeordnet ist, zwei freie Drahtverbindungen aufweist, die jeweils mit einer Schaltungskarte verbunden sind, welche den Hauptkern trägt.

10. Magnetfeldgenerator für eine elektromedizinische Behandlungsvorrichtung zur Behandlung von biologischem Gewebe, welcher mindestens eine Magnetspule (3) und einen Hauptkem (7) aufweist, wobei die Magnetspule auf dem Hauptkern angeordnet ist, in welchem eine Öffnung (111) definiert ist, sowie ein System magnetischer Linsen, welches eine gerade Anzahl von Linsenelementen (116 a - d) umfasst, die im Inneren der Öffnung in dem Hauptkern angeordnet sind, bei welchem jede magnetische Linse ein Linsenkernelement mit zwei freien Enden (115a, 115b) sowie eine Linsen-Magnetspule (16) aufweist, die auf dem Linsenkernelement angeordnet ist, **dadurch gekennzeichnet, dass** die Linsen-Magnetspulen, die auf dem Linsenkernelement angeordnet sind, jeweils zwei freie Drahtverbindungen (118a, 118b) aufweisen, von denen jede an einer Schaltungskarte (101) angeschlossen ist, welche als Träger des Hauptkerns fungiert.

11. Magnetfeldgenerator nach Anspruch 10, **dadurch gekennzeichnet, dass** der Magnetfeldgenerator des Weiteren ein Stützelement für die magnetischen Linsen aufweist, die in der Öffnung in dem Hauptkern angeordnet sind.

12. Magnetfeldgenerator nach Anspruch 11, **dadurch gekennzeichnet, dass** das Stützelement in der Öffnung des Hauptkerns in der Mitte angeordnet ist.

13. Magnetfeldgenerator nach einem der Ansprüche 11 - 12, **dadurch gekennzeichnet, dass** das Stützelement Wandungsabschnitte aufweist, welche eine Vertiefung bilden, die eine Verschiebung der Linsen-Magnetspulen verhindert.

14. Magnetfeldgenerator nach einem der Ansprüche 11 - 13, **dadurch gekennzeichnet, dass** das Stützelement Wandungsabschnitte aufweist, welche eine Vertiefung bilden, die ein Verschiebung des Linsenkerns in einer Richtung zur Mitte der Öffnung im Hauptkern hin verhindert.

15. Magnetfeldgenerator nach einem der Ansprüche 11 - 14, **dadurch gekennzeichnet, dass** die Vertiefung zusammen mit dem Hauptkernelement jedes Linsenelement umschließt und eine Verschiebung jedes Linsenelements in einer Ebene verhindert, welche durch das Hauptkernelement hindurch verläuft.

16. Magnetfeldgenerator nach einem der Ansprüche 11 - 15, **dadurch gekennzeichnet, dass** das Stützelement Verteilungskanäle aufweist, welche für die Einspritzung einer Verfestigungsmasse den Zugang schaffen.

17. Magnetfeldgenerator nach einem der Ansprüche 11 - 16, **dadurch gekennzeichnet, dass** das Stützelement auf einer Schaltungskarte angeordnet ist, welche als Träger des Hauptkerns fungiert.

18. Magnetfeldgenerator nach einem der Ansprüche 11 - 17, **dadurch gekennzeichnet, dass** das Stützelement einen Mittelträger für die Hauptspule bildet.

19. Feldgenerator für eine elektromedizinische Behandlungsvorrichtung zur Behandlung von biologischem Gewebe, welcher einen Magnetfeldgenerator mit mindestens einer Magnetspule (3) und einem Hauptkern aufweist, wobei die Magnetspule auf dem Hauptkern angeordnet ist, in welchem eine Öffnung (111) definiert ist, sowie eine gerade Anzahl von Linsenelementen (116 a - d), die im Inneren der Öffnung in dem Hauptkern angeordnet sind, bei welchem jede magnetische Linse ein Linsenkernelement (12 - 15) mit zwei freien Enden (115a, 115b) sowie eine Magnetspule (16) aufweist, die auf dem Linsenkernelement angeordnet ist, und welcher einen elektrischen Feldgenerator mit einem System elektrischer Linsen aufweist, das eine gerade Anzahl von Linsenelementen (19 .- 22) umfasst, welche im Inneren der Öffnung in dem Hauptkern radial angeordnet sind,
**dadurch gekennzeichnet, dass** die elektrischen Linsenelemente auf einer Schaltungskarte (101) angeordnet sind, welche den Träger des Hauptkerns bildet.

20. Feldgenerator nach Anspruch 19, **dadurch gekennzeichnet, dass** der Feldgenerator des Weiteren ein Stützelement für die magnetischen Linsen aufweist, die in der Öffnung im Hauptkern angeordnet sind.

21. Feldgenerator nach Anspruch 20, **dadurch gekennzeichnet, dass** der Feldgenerator des Weiteren ein Stützelement für die magnetischen Linsen aufweist, die in der Öffnung des Hauptkems angeordnet sind.

22. Feldgenerator nach Anspruch 20 oder 21, **dadurch gekennzeichnet, dass** das Stützelement in der Öffnung im Hauptkern in der Mitte angeordnet ist.

23. Feldgenerator nach einem der Ansprüche 20 - 22, **dadurch gekennzeichnet, dass** das Stützelement Wandungsabschnitte aufweist, welche eine Vertiefung bilden, die eine Verschiebung der Linsen-Magnetspulen verhindert.

24. Feldgenerator nach einem der Ansprüche 20 - 23, **dadurch gekennzeichnet, dass** das Stützelement Wandungsabschnitte aufweist, welche eine Vertiefung bilden, die eine Verschiebung des Linsenkerns in einer Richtung zur Mitte der Öffnung im Hauptkern hin verhindert.

25. Feldgenerator nach einem der Ansprüche 20 - 24, **dadurch gekennzeichnet, dass** die Vertiefung zusammen mit dem Hauptkernelement jedes Linsenelement umschließt und eine Verschiebung jedes Linsenelements in einer Ebene verhindert, welche durch das Hauptkernelement hindurch verläuft.

26. Feldgenerator nach einem der Ansprüche 20 - 25, **dadurch gekennzeichnet, dass** das Stützelement Verteilungskanäle aufweist, welche für die Einspritzung einer Verfestigungsmasse Zugang schaffen.

27. Feldgenerator nach einem der Ansprüche 20 - 26, **dadurch gekennzeichnet, dass** das Stützelement auf einer Schaltungskarte angeordnet ist, welche den Hauptkern trägt.

28. Feldgenerator nach einem der Ansprüche 20 - 27, **dadurch gekennzeichnet, dass** das Stützelement eine Mittelabstützung für die Hauptspule bildet.

29. Elektromagnetische Behandlungsvorrichtung zur Behandlung von biologischem Gewebe, vorzugsweise zur Beschleunigung des Heilungsprozesses in einem Teil des menschlichen Körpers wie beispielsweise einem Arm oder einem Bein, welches einen ersten und einen zweiten Feldgenerator nach einem der vorhergehenden Ansprüche aufweist, bei welchem der erste und der zweite Magnetfeldgenerator so angeordnet sind, dass sie ein magnetisches Wechselfeld erzeugen, und dass die elektromagnetische Behandlungsvorrichtung außerdem eine erste und eine zweite Elektrode aufweist, die zum Erzeugen eines elektrischen Feldes senkrecht zu dem Magnetfeld angeordnet sind.

30. Verfahren zur Herstellung eines Feldgenerators für eine elektromedizinische Behandlungsvorrichtung zur Behandlung von biologischem Gewebe nach einem der vorhergehenden Ansprüche, welches die folgenden Arbeitsgänge zur Herstellung umfasst:
- als erstes Anschließen von magnetischen Linsen, von einem Träger für magnetische Linsen und einem Hauptkernelement an eine Schattungskarte;
- danach Anordnen einer Hauptmagnetspule auf dem Hauptkern.

31. Verfahren zur Herstellung eines Feldgenerators nach Anspruch 30, **dadurch gekennzeichnet, dass** die Magnetspule eine axiale Abstützung für die magnetischen Linsenelemente bildet.

32. Verfahren zur Herstellung eines Feldgenerators nach Anspruch 30 oder 31, **dadurch gekennzeichnet, dass** die Magnetspule eine axiale Abstützung für die magnetischen Linsenelemente bildet.

33. Verfahren zur Herstellung eines Feldgenerators nach einem der Ansprüche 30 - 32, **dadurch gekennzeichnet, dass** der Feldgenerator in einem Gehäuse eingeschlossen wird.

34. Verfahren zur Herstellung eines Feldgenerators nach Anspruch 33, **dadurch gekennzeichnet, dass** in das Gehäuse eine Verfestigungsmasse eingespritzt wird.

## Revendications

1. Générateur de champ magnétique pour un appareil de traitement électromédical d'un tissu biologique, contenant au moins un solénoïde (3) et un noyau principal (7), le solénoïde étant disposé essentiellement de manière coaxiale, avec une ouverture (111) définie dans ledit noyau principal et un système à lentilles magnétiques comprenant un nombre pair d'éléments de lentille (114ad) disposés à l'intérieur de ladite ouverture dudit noyau principal, chaque lentille magnétique comportant un élément de noyau (12-15) de lentille ayant deux extrémités libres (115a, 115b) et un solénoïde (16) de lentille disposé sur ledit élément de noyau de lentille, ***caractérisé en ce que*** ledit générateur de champ magnétique comprend de plus un élément de support (102) pour les lentilles magnétiques installé dans ladite ouverture dudit noyau principal.

2. Générateur de champ magnétique selon la revendication 1, ***caractérisé en ce que*** ledit élément de support est installé centralement dans ladite ouverture dudit noyau principal.

3. Générateur de champ magnétique selon la revendication 1 ou 2, ***caractérisé en ce que*** ledit élément de support comprend des portions de paroi formant un évidement empêchant le déplacement des solénoïdes des lentilles.

4. Générateur de champ magnétique selon l'une quelconque des revendications 1 à 3, ***caractérisé en ce que*** ledit élément de support comprend des portions de paroi formant un évidement empêchant le déplacement du noyau des lentilles en direction du centre de l'ouverture dudit noyau principal.

5. Générateur de champ magnétique selon l'une quelconque des revendications 1 à 4, ***caractérisé en ce que*** ledit évidement, de concert avec l'élément de noyau principal, entoure chaque élément de lentille et empêche chaque élément de lentille de se déplacer dans un plan traversant l'élément de noyau principal.

6. Générateur de champ magnétique selon l'une quelconque des revendications 1 à 5, ***caractérisé en ce que*** ledit élément de support comporte des goulottes de distribution destinées à offrir un accès pour l'injection d'un composé se solidifiant.

7. Générateur de champ magnétique selon l'une quelconque des revendications 1 à 6, ***caractérisé en ce que*** ledit élément de support est agencé de manière à supporter une carte imprimée supportant ledit noyau principal.

8. Générateur de champ magnétique selon l'une quelconque des revendications 1 à 7, ***caractérisé en ce que*** ledit élément de support fournit un support à la bobine principale.

9. Générateur de champ magnétique selon l'une quelconque des revendications 1 à 8, ***caractérisé en ce que*** chaque dit solénoïde de lentille installé sur ledit élément de noyau de lentille possède deux fils de connexion libres, raccordé chacun à une carte imprimée supportant ledit noyau principal.

10. Générateur de champ magnétique pour un appareil de traitement électromédical d'un tissu biologique, contenant au moins un solénoïde (3) et un noyau principal (7), le solénoïde étant disposé sur ledit noyau principal en définissant une ouverture (111), et un système à lentilles magnétiques comprenant un nombre pair d'éléments de lentille (114a-d) disposés à l'intérieur de ladite ouverture dudit noyau principal, chaque lentille magnétique comportant un élément de noyau de lentille ayant deux extrémités libres (115a, 115b) et un solénoïde de lentille disposé sur ledit élément de noyau de lentille, ***caractérisé en ce que*** les solénoïdes de lentille disposés sur les éléments de noyau de lentille ont chacun deux fils de connexion libres (118a, 118b) raccordé chacun à une carte imprimée (101) supportant ledit noyau principal.

11. Générateur de champ magnétique selon la revendication 10, ***caractérisé en ce que*** ledit générateur de champ magnétique comprend en plus un élément de support pour les lentilles magnétiques, installé dans ladite ouverture dudit noyau principal.

12. Générateur de champ magnétique selon la revendication 11, ***caractérisé en ce que*** ledit élément de support est installé centralement dans ladite ouverture dudit noyau principal.

13. Générateur de champ magnétique selon l'une quelconque des revendications 11 et 12, ***caractérisé en ce que*** ledit élément de support comprend des portions de paroi formant un évidement empêchant le déplacement des solénoïdes des lentilles.

14. Générateur de champ magnétique selon l'une quelconque des revendications 11 à 13, ***caractérisé en ce que*** ledit élément de support comprend des portions de paroi formant un évidement empêchant le déplacement du noyau des lentilles en direction du centre de l'ouverture dudit noyau principal.

15. Générateur de champ magnétique selon l'une quelconque des revendications 11 à 14, ***caractérisé en ce que*** ledit évidement, de concert avec l'élément de noyau principal, entoure chaque élément de lentille et empêche chaque élément de lentille de se déplacer dans un plan traversant l'élément de noyau principal.

16. Générateur de champ magnétique selon l'une quelconque des revendications 11 à 15, ***caractérisé en ce que*** ledit élément de support comporte des goulottes de distribution destinées à offrir un accès pour l'injection d'un composé se solidifiant.

17. Générateur de champ magnétique selon l'une quelconque des revendications 11 à 16, ***caractérisé en ce que*** ledit élément de support est agencé de manière à supporter une carte imprimée supportant ledit noyau principal.

18. Générateur de champ magnétique selon l'une quelconque des revendications 11 à 17, ***caractérisé en ce que*** ledit élément de support fournit un support central à la bobine principale.

19. Générateur de champ pour un appareil de traitement électromédical d'un tissu biologique, comportant un générateur de champ magnétique ayant au moins un solénoïde (3) et un noyau principal, le solénoïde étant disposé sur ledit noyau principal en définissant une ouverture (111), et un système à lentilles magnétiques comprenant un nombre pair d'éléments de lentille (114a-d) disposés à l'intérieur de ladite ouverture dudit noyau principal, chaque lentille magnétique comportant un élément de noyau (12-15) de lentille ayant deux extrémités libres (115a, 115b) et un solénoïde (16) disposé sur ledit élément de noyau de lentille, et un générateur de champ électrique ayant un système de lentilles électriques comprenant un nombre pair d'éléments de lentille (19-22) disposés radialement dans l'ouverture dudit noyau principal, ***caractérisé en ce que*** les éléments de lentille électrique sont installés sur une carte imprimée (101) supportant ledit noyau principal.

20. Générateur de champ selon la revendication 19, ***caractérisé en ce que*** ledit générateur de champ comprend en plus un élément de support pour les lentilles magnétiques, disposé dans ladite ouverture dudit noyau principal.

21. Générateur de champ selon la revendication 20, ***caractérisé en ce que*** ledit générateur de champ comprend en plus un élément de support pour les lentilles magnétiques, disposé dans ladite ouverture dudit noyau principal.

22. Générateur de champ selon la revendication 20 ou 21, ***caractérisé en ce que*** ledit élément de support est disposé centralement dans ladite ouverture dudit noyau principal.

23. Générateur de champ selon l'une quelconque des revendications 20 à 22, ***caractérisé en ce que*** ledit élément de support comprend des portions de paroi formant un évidement empêchant le déplacement des solénoïdes des lentilles.

24. Générateur de champ selon l'une quelconque des revendications 20 à 23, ***caractérisé en ce que*** ledit élément de support comprend des portions de paroi formant un évidement empêchant le déplacement du noyau des lentilles en direction du centre de l'ouverture dudit noyau principal.

25. Générateur de champ selon l'une quelconque des revendications 20 à 24, ***caractérisé en ce que*** ledit évidement, de concert avec l'élément de noyau principal, entoure chaque élément de lentille et empêche chaque élément de lentille de se déplacer dans un plan traversant l'élément de noyau principal.

26. Générateur de champ selon l'une quelconque des revendications 20 à 25, ***caractérisé en ce que*** ledit élément de support comporte des goulottes de distribution destinées à offrir un accès pour l'injection d'un composé se solidifiant.

27. Générateur de champ magnétique selon l'une quelconque des revendications 20 à 26, ***caractérisé en ce que*** ledit élément de support est installé sur une carte imprimée supportant ledit noyau principal.

28. Générateur de champ magnétique selon l'une quelconque des revendications 20 à 27, ***caractérisé en ce que*** ledit élément de support fournit un support central à la bobine principale.

29. Appareil de traitement électromagnétique destiné au traitement d'un tissu biologique, de manière préférée pour accélérer le processus de guérison dans une partie du corps humain telle qu'un bras ou une jambe, comprenant un premier et un deuxième générateurs magnétiques selon l'une quelconque des revendications précédentes, et dans lequel lesdits premier et deuxième générateurs magnétiques sont agencés de manière à fournir un champ magnétique alternatif et dans lequel ledit appareil de traitement électromagnétique comprend de plus une première et une deuxième électrodes agencées de manière à générer un champ électrique perpendiculaire audit champ magnétique.

30. Procédé de production d'un générateur de champ pour un appareil de traitement électromédical destiné au traitement d'un tissu biologique selon l'une quelconque des revendications précédentes, comprenant les étapes de production suivantes :
en premier lieu le raccordement de lentilles magnétiques, d'un support pour les lentilles magnétiques et d'un élément de noyau principal à une carte imprimée ;
- et ensuite l'agencement d'un solénoïde principal sur ledit noyau principal.

31. Procédé de production d'un générateur de champ selon la revendication 30, ***caractérisé en ce que*** le solénoïde fournit un support axial aux éléments de lentilles magnétiques.

32. Procédé de production d'un générateur de champ selon la revendication 30 ou 31, ***caractérisé en ce que*** le solénoïde fournit un support axial aux éléments de lentilles magnétiques.

33. Procédé de production d'un générateur de champ selon l'une quelconque des revendications 30 à 32, ***caractérisé en ce que*** le générateur de champ est enfermé dans un boîtier.

34. Procédé de production d'un générateur de champ selon l'une quelconque des revendications 33, ***caractérisé en ce qu****'un* composé se solidifiant est injecté dans le boîtier.
